(19) Europäisches Patentamt
European Patent Office
Office européen des brevets

(11) Veröffentlichungsnummer: **0 183 042**
**B1**

(12) # EUROPÄISCHE PATENTSCHRIFT

(45) Veröffentlichungstag der Patentschrift: **11.10.89**

(21) Anmeldenummer: **85113322.3**

(22) Anmeldetag: **21.10.85**

(51) Int. Cl.[4]: **C 07 C 69/18**, C 07 C 67/297, C 07 C 43/23, C 07 C 41/03, C 07 C 67/29// C07D311/72, C07D303/20, C07C39/19

(54) **Verfahren zur Herstellung von Hydrochinonderivaten.**

(30) Priorität: **21.11.84 CH 5558/84**
**11.09.85 CH 3927/85**

(43) Veröffentlichungstag der Anmeldung: **04.06.86 Patentblatt 86/23**

(45) Bekanntmachung des Hinweises auf die Patenterteilung: **11.10.89 Patentblatt 89/41**

(84) Benannte Vertragsstaaten: **AT BE CH DE FR GB IT LI NL**

(56) Entgegenhaltungen:
**FR–A– 863 306**
**FR–A– 2 208 881**

(73) Patentinhaber: **F. HOFFMANN-LA ROCHE AG**
**Postfach 3255**
**CH-4002 Basel (CH)**

(72) Erfinder: **Imfeld, Marquard, Dr.**
**Benkenstrasse 76**
**CH-4102 Binningen (CH)**

(74) Vertreter: **Zimmermann, Hans, Dr. et al**
**Grenzacherstrasse 124 Postfach 3255**
**CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 183 042 B1

**Beschreibung**

Die vorliegende Erfindung betrifft ein neues Verfahren zur Herstellung von Hydrochinonderivaten, welche als Zwischenprodukte für die Herstellung von (R,R,R)-α-Tocopherol (natürlichem Vitamin E) sowie von racemischem α-Tocopherol geeignet sind. Ferner betrifft die Erfindung die neuen Produkte dieses Verfahrens.

Es sind bereits einige Verfahren zur Herstellung von natürlichem Vitamin E bekannt, welche jedoch technisch nur von begrenztem Interesse sind, so dass natürliches Vitamin E bis heute nahezu ausschliesslich aus natürlichen Quellen extrahiert wird. Es bestand somit ein Bedürfnis nach einem technisch realisierbaren Zugang, gemäss welchem natürliches Vitamin E in guter Ausbeute und mit hoher optischer Reinheit erhalten werden kann.

Das erfindungsgemässe Verfahren ist dadurch gekennzeichnet, dass man eine Verbindung der allgemeinen Formel

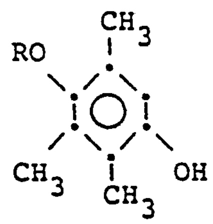

(I)

worin R eine Hydroxylschutzgruppe bedeutet, mit 3,4-Epoxy-3-methyl-1-buten in Gegenwart eines $d^{10}$-Uebergangsmetall-Katalysators umsetzt und gewünschtenfalls eine so erhaltene Verbindung der allgemeinen Formel

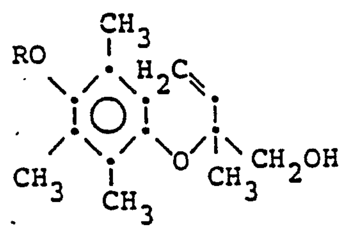

(II)

worin R die oben angegebene Bedeutung besitzt, einer Claisen-Umlagerung unterwirft, wobei eine Verbindung der allgemeinen Formel

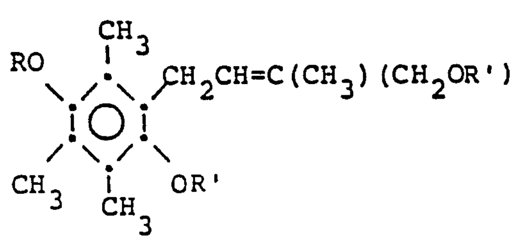

(III)

worin R die oben angegebene Bedeutung besitzt, und R' Wasserstoff oder eine Acylgruppe bedeutet, erhalten wird.

Der Ausdruck « Hydroxylschutzgruppe » bedeutet im Rahmen der vorliegenden Erfindung eine abspaltbare Gruppe, und zwar sowohl eine durch Hydrolyse abspaltbare Gruppe, wie die Silylgruppe, eine Alkoxymethylgruppe, z. B. Methoxymethyl, die Tetrahydropyranylgruppe oder eine Acylgruppe, z. B. Acetyl, als auch eine oxidativ abspaltbare Gruppe, wie eine $C_{1-6}$-Alkylgruppe, z. B. Methyl. Die bevorzugte Hydroxylschutzgruppe R ist Acetyl.

Der Ausdruck « Acylgruppe » bedeutet übliche Acylreste von aliphatischen und aromatischen Carbonsäuren, vorzugsweise solche mit bis zu 10 Kohlenstoffatomen, wie Acetyl, Propionyl, Benzoyl und dergleichen.

Die Formel III soll das cis-Isomere (Z-Form), das trans-Isomere (E-Form) als auch Gemische der cis- und der trans-Form umfassen.

Schliesslich bedeutet der Ausdruck « $d^{10}$-Uebergangsmetall-Katalysator » einen Uebergangsmetallkomplex, dessen zentrales Metallatom die $d^{10}$-Konfiguration aufweist und wobei die Liganden je nach Oxidationsgrad des Metallatoms ungeladen und/oder negativ geladen sind. Der ganze Komplex ist also ungeladen. Die in Frage kommenden Zentralatome sind Nickel(O), Kupfer(I), Palladium(O), Silber(I), Platin(O) und Gold(I) (siehe J.P. Collman und L.S. Hegedus, « Principles and Applications of Organotransition Metal Chemistry », University Science Books, Mill Valley, California, 1980, Seite 13ff). Beispiele geeigneter Liganden sind Triorganophosphin, wie Triphenylphosphin oder Trimethylphosphin ; Kohlenmonoxid ; ein aromatischer Ligand, wie Benzol ; Halogenid, wie Chlorid ; und Sulfonat, wie Trifluormethansulfonat. Als Beispiele geeigneter Katalysatoren seien genannt :

Bis-(triphenylphosphin)-nickel(O)-dicarbonyl (siehe Merck Index 9, 1327),

Der Kupfer(I)-trifluormethansulfonat-Benzol-Komplex [siehe Tetrahedron Letters No. 27, 2529-2532 (1973)],

Tetrakis-(triphenylphosphin)-palladium(O) und die Verbindung der Formel $Pd^{o}$ $(diop)_2$, worin « diop » ein optisch aktiver Phosphinligand ist, und zwar (2S,3S)-2,3-Isopropyliden-2,3-dihydroxy-1,4-bis-(diphenylphosphino)-butan [(+)-diop] bzw. (2R,3R)-2,3-O-isopropyliden-2,3-dihydroxy-1,4-bis-(diphenylphosphino)-butan [(-)-diop] [siehe z. B. Tetrahedron 33, 2615-2649 (1977) und Topics in Stereochemistry 10, 175-285 (1978)],

(Trimethylphosphin)-silber(I)-chlorid [siehe z. B. Chem. Ber. 105, 3382-3388 (1972)],

Tetrakis-(triphenylphosphin)-platin(O) [siehe z. B. Inorganic Synthesis 11, 105-108 (1968)] und

(Triphenylphosphin)-gold(I)-chlorid [siehe z. B. Chemistry and Industry 1959, 1628].

Im Falle eines Palladium(O)-Katalysators kann dieser beispielsweise aus einem Palladium(II)-Salz im Reaktionsgemisch in situ durch Reduktion erzeugt werden, z. B. aus Palladium(II)-acetat in Gegenwart eines Reduktionsmittels, wie Ameisensäure, Hydrazinhydrat, eines tertiären Amins, z. B. Triäthylamin, oder eines aliphatischen oder cyclischen Aethers, z. B. Tetrahydrofuran, und einer mit Palladium(O) Ligand-bildenden Verbindung, z. B. Triphenylphosphin.

Die Palladium(O)-enthaltenden Komplexe sind die bevorzugten $d^{10}$-Uebergangsmetall-Katalysatoren.

Die Umsetzung der Verbindung der Formel I mit 3,4-Epoxy-3-methyl-1-buten erfolgt zweckmässigerweise in einem inerten Verdünnungsmittel, insbesondere einem organischen Lösungsmittel, wie einem aliphatischen oder cyclischen Aether, z. B. Diäthyläther, Diisopropyläther oder Tetrahydrofuran ; einem aromatischen Kohlenwasserstoff, z. B. Benzol, Toluol oder einem Xylol ; oder einem halogenierten aliphatischen Kohlenwasserstoff, z. B. Methylenchlorid oder Chloroform. Im Falle von Aethern wirken diese oft auch als Reduktionsmittel zur Ueberführung von eingesetztem Palladium(II) in Palladium (O). So haben sich beispielsweise Diäthyläther und Tetrahydrofuran sowohl als geeignete Lösungsmittel als auch als geeignete Reduktionsmittel in jenen erfindungsgemässen Verfahren gezeigt, in denen Palladium(II)-Salze eingesetzt wurden.

Diese Umsetzung wird im allgemeinen bei Temperaturen zwischen 0° und 30 °C, vorzugsweise bei Raumtemperatur, durchgeführt.

Die Menge des vorhandenen $d^{10}$-Uebergangsmetall-Katalysators bzw. Palladium(II)-Salzes beträgt im allgemeinen mindestens 0,01, insbesondere 0,05 bis 100 Molprozent, bezogen auf die Menge Verbindung der Formel I, vorzugsweise ca. 0,3 Molprozent.

Die fakultative Ueberführung der Verbindung der Formel II in die Verbindung der Formel III kann in allgemeinen unter den an sich bekannten Bedingungen von Claisen-Umlagerungen durchgeführt werden. So erfolgt die erfindungsgemässe Umlagerung zweckmässigerweise in einem inerten Verdünnungsmittel, insbesondere einem organischen Lösungsmittel, wie einem aliphatischen oder aromatischen Kohlenwasserstoff, z. B. n-Heptan, Benzol, Toluol oder einem Xylol ; einem Heteroaromaten, z. B. Pyridin ; einem halogenierten aliphatischen oder aromatischen Kohlenwasserstoff, z. B. Methylenchlorid, Chloroform oder Chlorbenzol ; einem aliphatischen oder cyclischen Aether, z. B. Diäthyläther, Tetrahydrofuran oder Dioxan ; einem Ester, z. B. Essigsäureäthylester ; einem nitrierten Lösungsmittel, z. B. Nitromethan ; oder einem Formamid, z. B. Dimethylformamid. Die Reaktionstemperaturen können in einem grossen Bereich, wie — 10 °C bis 140 °C, variiert werden.

Temperaturen, d. h. im Temperaturbereich — 10° bis + 30 °C, wobei zweckmässigerweise entweder in Gegenwart einer Protonensäure, wie Essigsäure, Salzsäure, Trifluoressigsäure, Phosphorsäure oder Schwefelsäure, in Gegenwart eines Carbonsäureanhydrids oder -halogenids, z. B. Acetanhydrid oder Acetylchlorid, oder aber in Gegenwart einer Lewissäure, wie Eisen(III)-chlorid oder Bortrifluorid oder eines Komplexes einer solchen Säure, z. B. deren Aetherate, gearbeitet wird, oder bei erhöhten Temperaturen, d. h. im Temperaturbereich 80 °C bis 140 °C, insbesondere bei ca. 100 °C, wobei gewünschtenfalls in Gegenwart einer Base, wie Pyridin, Piperidin oder Dimethylaminopyridin, gearbeitet wird.

Die Claisen-Umlagerung in Gegenwart eines Carbonsäureanhydrids oder eines Carbonsäurehalogenids führt zu einer Verbindung der Formel III, worin R' eine Acylgruppe bedeutet. In den übrigen Fällen wird eine Verbindung der Formel III erhalten, worin R' Wasserstoff bedeutet.

In der Regel liegt das Produkt der oben beschriebenen Umlagerung eher in Form eines Gemisches der isomeren Verbindungen der allgemeinen Formeln

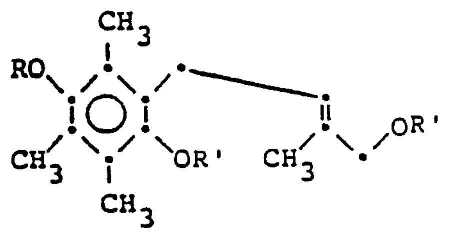

(IIIa)

(E-Form)

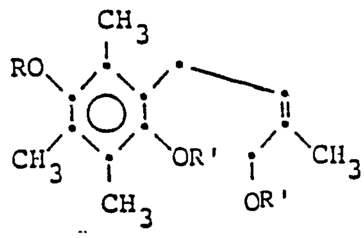

(IIIb)

(Z-Form)

vor, als ausschliesslich in Form des einen oder des anderen Isomeren. Je nach den Reaktionsbedingungen der Umlagerung beträgt das Verhältnis E-Form(IIIa) : Z-Form(IIIb) insbesondere von 50 : 50 bis fast 100 : 0. Es wurde beispielsweise gefunden, dass bei der Durchführung der Umlagerung in saurem Medium, z. B. unter Verwendung von Chlorwasserstoff in Methylenchlorid, fast ausschliesslich die E-Form des Produktes (das trans-Isomere) erhalten wird. Im basischen Medium, z. B. unter Verwendung von Dimethylaminopyridin in Toluol, liefert hingegen die Umlagerung einen wesentlichen Anteil der Z-Form des Produktes (das cis-Isomere), wobei jedoch ein erhöhtes cis(Z) : trans(E)-Verhältnis durch verminderten Einsatz der Base begünstigt wird.

Die Isolierung und die Reinigung der so hergestellten Verbindungen der Formeln II und III können nach an sich bekannten Methoden erfolgen. Die reine E-Form und die reine Z-Form der Verbindung der Formel III, d. h. die reine Verbindung der Formel IIIa und die reine Verbindung der Formel IIIb, sind durch an sich bekannte Methoden, z. B. Säulenchromatographie, isolierbar.

Die Verbindungen der allgemeinen Formeln II, III, IIIa und IIIb sind neu und, wie oben gesagt, ebenfalls Gegenstand der vorliegenden Erfindung. In diesen Formeln sind R und R′ vorzugsweise Acetyl.

Die Verbindungen der allgemeinen Formeln III, IIIa und IIIb können in (R,R,R)-α-Tocopherol (natürliches Vitamin E) übergeführt werden, beispielsweise gemäss dem nachfolgenden Reaktionsschema 1. Es wird dabei von der E-Form (Verbindung der Formel IIIa) ausgegangen, jedoch gelten die Reaktionsstufen auch im Falle des Isomerengemisches (Verbindungen der Formeln IIIa und IIIb) oder der Z-Form (Verbindung der Formel IIIb) als Ausgangsmaterial. Das Verfahren führt in jedem Fall zum gewünschten Isomeren, d. h. zum (R,R,R)-α-Tocopherol. In diesem Reaktionsschema bedeutet das ausgefüllte keilförmige Zeichen, dass sich der entsprechende Rest oberhalb der Molekülebene befindet, während die gestrichelte Linie bedeutet, dass sich der entsprechende Rest unterhalb der Molekülebene befindet.

(Siehe Formeln Seite 5 ff.)

Die Ueberführung der Verbindung der Formel IIIaa in die Verbindung der Formel IV (Abspaltung der Hydroxyschutzgruppe R) kann je nach Art der Schutzgruppe R unter den diesbezüglichen Reaktionsbedingungen durchgeführt werden. Falls die Schutzgruppe R durch Hydrolyse abspaltbar ist, kann dies in einfacher Weise, beispielsweise durch Behandlung mit einer Säure (R bedeutet z. B. Silyl, Alkoxymethyl oder Tetrahydropyranyl) bzw. mit einer Base (R bedeutet z. B. Acyl), erfolgen. Fals die Schutzgruppe R oxidativ abspaltbar ist, erfolgt die Ueberführung auch in einfacher Weise, z. B. durch Behandlung mit Cerammoniumnitrat $[(Ce(NH_4)_2(NO_3)_6]$ und anschliessende reduktive Cyclisierung des erhaltene Chinons.

Auch die selektive O-Methylierung der Verbindung der Formel IV zur Verbindung der Formel V kann unter an sich bekannten Reaktionsbedingungen durchgeführt werden, z. B. unter Phasentransferbedingungen unter Verwendung von Dimethylsulfat als Methylierungsmittel, von Methylenchlorid/Wasser als Lösungsmittel, einer Base, wie Natrium- oder Kaliumhydroxid, und eines Phasentransferkatalysators, wie Tetrabutylammoniumbromid.

Die anschliessende Sharpless-Epoxidierung der Verbindung der Formel V zur Verbindung der Formel VI ist eine an sich bekannte Reaktion und kann unter den üblichen Bedingungen durchgeführt werden, z. B. unter Verwendung von tert.-Butylhydroperoxid als Oxidationsmittel in Gegenwart von Titantetraisopropoxid und Dibutyl-D-tartrat in Methylenchlorid im Temperaturbereich von —20 °C bis zur Raumtemperatur.

Die Reduktion der Verbindung der Formel VI zur Verbindung der Formel VII kann unter Verwendung von Wasserstoff in Gegenwart von Raney-Nickel oder mit Lithiumaluminiumhydrid durchgeführt werden. Die erstgenannte Methode erfolgt zweckmässigerweise in einem wässrigen organischen Lösungsmittel, wobei geeigneterweise als organischer Teil des Lösungsmittels ein mit Wasser mischbares organisches Lösungsmittel in Frage kommt. Bevorzugte organische Lösungsmittel sind niedere Alkanole, wie Methanol, Aethanol und Propanol ; aliphatische oder cyclische Aether, wie Diäthyläther, Tetrahydrofuran und Dioxan ; oder Ketone, wie Aceton. Die Reduktion erfolgt weiterhin zweckmässig in neutralem bis schwach alkalischem pH-Bereich, insbesondere im pH-Bereich von etwa 7-10, und bei Temperaturen von

Reaktionsschema 1

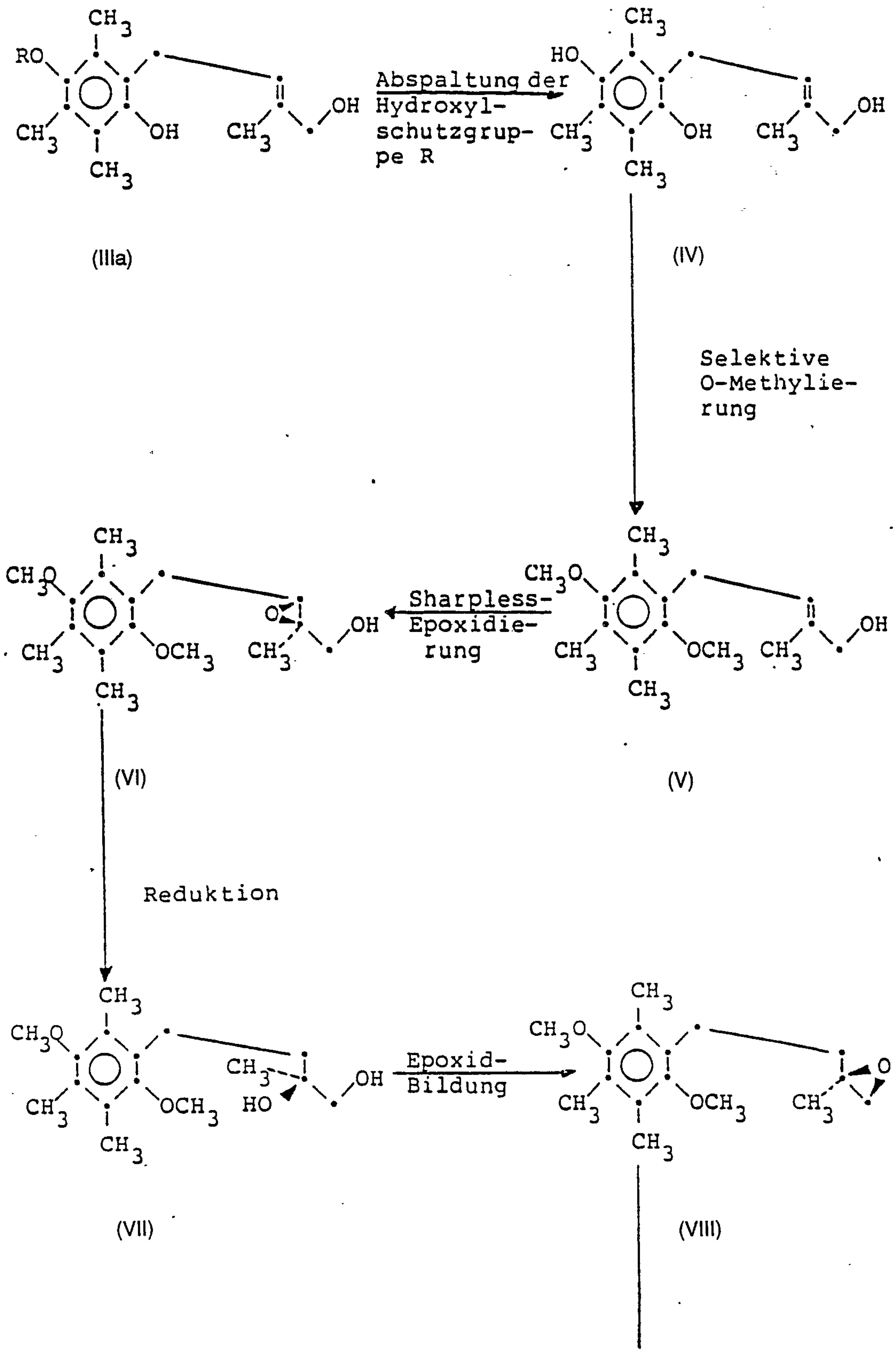


(IIIa)

(IV)

(V)

(VI)

(VII)

(VIII)

(Fortsetzung)

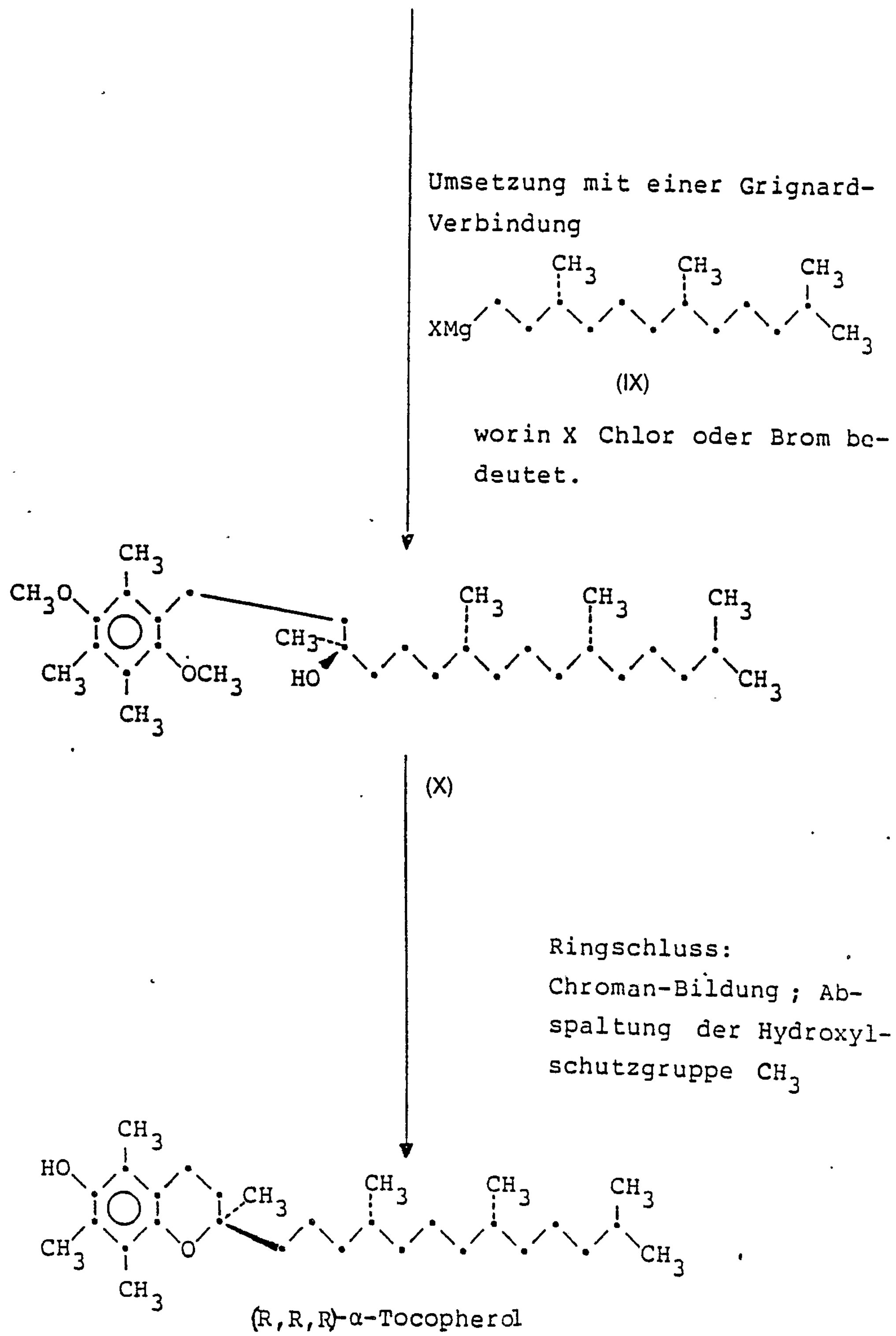


(IX)

(X)

(R,R,R)-α-Tocopherol

(XI)

etwa 70 °C bis etwa 100 °C, vorzugsweise bei der Rückflusstemperatur der Reaktionsgemisches. Besonders bevorzugt ist ein Lösungsmittelgemisch, dessen Siedepunkt möglichst nahe bei 100 °C liegt. Die Reduktion mit Lithiumaluminiumhydrid erfolgt zweckmässigerweise in einem aliphatischen oder cyclischen Aether, wie Diäthyläther, Tetrahydrofuran oder Dioxan, und bei Raumtemperatur.

Die Ueberführung der Verbindung der Formel VII in das Epoxid der Formel VIII kann in an sich

bekannter Weise durchgeführt werden. Zu diesem Zweck wird in eine Verbindung der Formel VII zunächst die primäre Hydroxygruppe in eine Abgangsgruppe übergeführt, z. B. in ein Halogenid (als Halogen kommt hier Chlor, Brom oder Jod in Frage) oder in einen Sulfonsäureester (z. B. Tosylat oder Mesylat) und dergleichen. Dies kann in an sich bekannter weise erfolgen. Anschliessend wird die so erhaltene Verbindung mit einer Base behandelt, und zwar auch in an sich bekannter Weise. Als Basen eignen sich sowohl anorganische als auch organische Basen, vorzugsweise jedoch anorganische Basen, wie insbesondere Natriumhydroxid, Kaliumhydroxid und dergleichen.

Auch die Umsetzung eines Epoxids der Formel VIII mit einer Grignard-Verbindung der Formel IX kann in an sich bekannter Weise durchgeführt werden. Bevorzugt ist jedoch die Umsetzung in Gegenwart eines Kupfer (I oder II)-Katalysators, insbesondere Kupfer(I)-n-Propylacetylid oder eines Kupfer(I)-halogenid-dimethylsulfid-Komplexes. Als Lösungsmittel eignen sich für diese Umsetzung alle bei Grignard-Reaktionen üblicherweise in Frage kommenden Lösungsmittel.

Die Verbindung der Formel X ist bekannt und kann in bekannter Weise in (R,R,R)-α-Tocopherol (XI) übergeführt werden. Dies kann beispielsweise in einfacher Weise durch Behandlung mit z. B. Cerammoniumnitrat $[(Ce(NH_4)_2—(NO_3)_6]$ und anschliessende reduktive Cyclisierung des erhaltene Chinons erfolgen.

Die Verbindungen der Formel III, worin R′ eine Acylgruppe bedeutet, können nach selektiver Abspaltung der Acylgruppe in der Seitenkette in analoger Weise zur Verbindung der Formel V weiter umgesetzt werden. Die Abspaltung der Acylgruppe kann nach an sich bekannten Methoden erfolgen, beispielsweise in Gegenwart von Bortrifluorid, Titan-(IV)-salzen, Triäthylamin oder Natriumhydroxid.

Die Verbindung der allgemeinen Formel III kann aber auch in racemisches α-Tocopherol übergeführt werden, beispielsweise gemäss dem nachfolgenden Reaktionsschema 2.

(Siehe Schema Seite 8 f.)

Die Ueberführung der Verbindung der Formel IIIc in die Verbindung der Formel XII (Einführung einer Abgangsgruppe Y) kann je nach Art der Abgangsgruppe Y unter den diesbezüglichen an sich bekannten Reaktionsbedingungen durchgeführt werden, z. B. durch Behandlung der Verbindung der Formel III mit Methan- bzw. p-Toluolsulfonsäurechlorid in Pyridin. Auch die anschliessende Umsetzung der Verbindung der Formel XII mit der Verbindung der Formel IX′ kann unter an sich bekannten Reaktionsbedingungen erfolgen. Die zum racemischen α-Tocopherol führende letzte Reaktionsstufe erfolgt zweckmässigerweise in einem organischen Verdünnungsmittel, vorzugsweise Toluol, Essigsäure, 1,2-Dichloräthan oder n-Heptan, und in Gegenwart einer wässrigen Mineralsäure, wie Salzsäure oder Bromwasserstoffsäure, und/oder in Gegenwart einer Lewissäure, wie Aluminiumtrichlorid oder Bortrifluorid.

Die Isolierung und die Reinigung sämtlicher in den obigen Reaktionsschemata 1 und 2 dargestellten Reaktionsprodukte können nach an sich bekannten Methoden durchgeführt werden.

Das erfindungsgemässe Verfahren wird durch die nachstehenden Beispiele 1, 2 und 10 illustriert, während die stufenweise Ueberführung des Produktes der Formel III in (R,R,R)-α-Tocopherol durch die Beispiele 3-9 und 11 illustriert wird.

## Beispiel 1

Herstellung von 2,3,6-Trimethyl-4-(1′-hydroxymethyl-1′-methyl-allyloxy)-phenylacetat

a) Zu einer Lösung von 10 g 2,3,6-Trimethylhydrochinon-1-monoacetat (Formel I, $R = CH_3CO$) in 40 ml Methylenchlorid werden bei Raumtemperatur 120 mg Tetrakis-(triphenylphosphin)-palladium(O), anschliessend 5,0 g 3,4-Epoxy-3-methyl-1-buten, gegeben. Nach Rühren während 30 Minuten bei Raumtemperatur wird das Reaktionsgemisch mit wässriger Natriumbicarbonatlösung gewaschen und dann die organische Phase über wasserfreiem Natriumsulfat getrocknet und eingedampft. Aus dem Rückstand werden mittels Säulenchromatographie (Kieselgel, Diäthyläther/n-Hexan) 12 g reines Produkt, 2,3,6-Trimethyl-4-(1′-hydroxymethyl-1′-methyl-allyloxy)-phenylacetat, in Form eines farblosen Oels gewonnen.

b) Die Methode von a) wird wiederholt, jedoch mit den Unterschieden, dass als Lösungsmittel Diäthyläther verwendet wird, und dass anstelle von Tetrakis-(triphenylphosphin)-palladium(O) Palladium (II)-acetat (116 mg) und Triphenylphosphin (540 mg) eingesetzt werden. In diesem Fall wird also der Palladium(O)-Katalysator in situ erzeugt. Man erhält das gleiche Produkt wir dasjenige der Methode a)

c) Die Methode von a) wird wiederholt, jedoch mit dem Unterschied, dass anstelle von Tetrakis-(triphenylphosphin)-palladium(O) Palladium(II)-acetat (116 mg), Triphenylphosphin (680 mg) und Ameisensäure (50 mg, Reduktionsmittel) eingesetzt werden. Auch in diesem Fall wird also der Palladium(O)-Katalysator in situ erzeugt. Man erhält das gleiche Produkt wir dasjenige der Methode a).

d) Die Methode von a) wird wiederholt, jedoch mit den Unterschieden, dass als Katalysator der Kupfer(I)-trifluormethansulfonat-Benzol-Komplex (40 mg) verwendet und 4 Stunden gerührt wird. Man erhält das gleiche Produkt wie in a).

Reaktionsschema 2

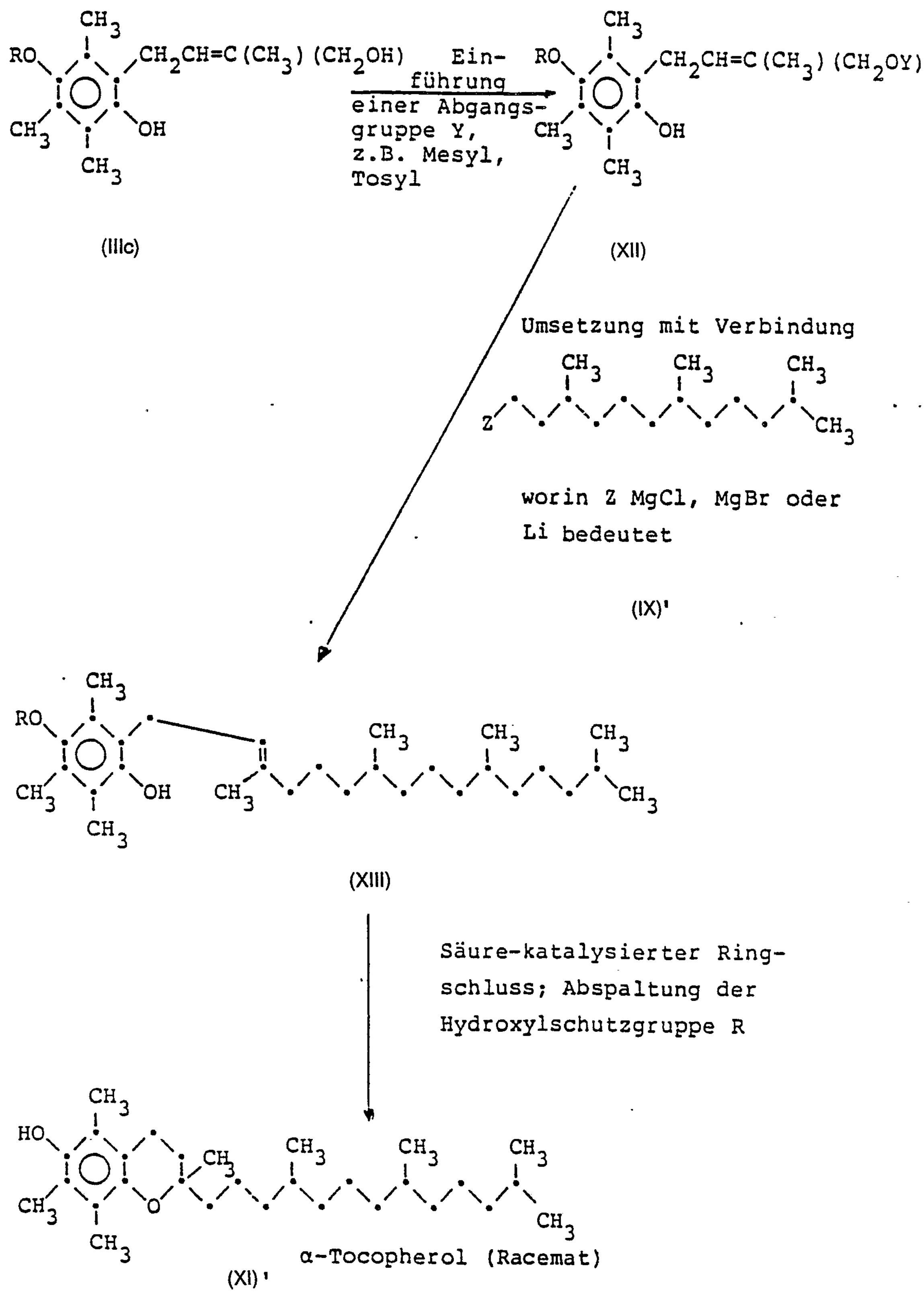


(IIIc)

(XII)

(IX)'

(XIII)

α-Tocopherol (Racemat)

(XI)'

e) Die Methode von a) wird wiederholt, jedoch mit den Unterschieden, dass als Katalysator Bis-(triphenylphosphin)-nickel(O)-dicarbonyl (980 mg) verwendet und ca. 16 Stunden bei Rückflusstemperatur erhitzt wird. Man erhält das gleiche Produkt wie in a).

f) Die Methode von a) wird wiederholt, jedoch mit dem Unterschied, dass als Katalysator Tetrakis-(triphenylphosphin)-platin(O) (180 mg) verwendet wird. Man erhält das gleiche Produkt wie in a).

Beispiel 2

Herstellung von 4-Hydroxy-5-(4′-hydroxy-3′-methyl-2′-butenyl)-2,3,6-trimethyl-phenylacetat

a) In eine Lösung von 5 g 2,3,6-Trimethyl-4-(1′-hydroxymethyl-1′-methyl-allyloxy)-phenylacetat in 80 ml Methylenchlorid wird bei 0 °C ein schwacher Chlorwasserstoff-Strom geleitet. Nach Rühren während 30 Minuten bei 0 °C wird die resultierende Suspension auf wässrige Natriumbicarbonatlösung gegossen. Die organische Phase wird über wasserfreiem Natriumsulfat getrocknet und eingedampft, und aus dem Rückstant (4,9 g) werden mittels Säulenchromatographie (Kieselgel, Diäthyläther/n-Hexan) 4,3 g 4-Hydroxy-5-(4′-hydroxy-3′-methyl-2′-butenyl)-2,3,6-trimethyl-phenylacetat in der E-Form, Smp. 144-146 °C, gewonnen.

b) Eine Lösung von 6,3 g 2,3,6-Trimethyl-4′-(1′-hydroxymethyl-1′-methyl-allyloxy)-phenylacetat in 150 ml Toluol wird während 6 Stunden bei Rückflusstemperatur erhitzt. Danach wird das Reaktionsgemisch eingedampft und der resultierende Rückstand chromatographiert (Kieselgel, Diäthyläther/n-Hexan/Methanol). Es Werden 4,7 g 4-Hydroxy-5-(4′-hydroxy-3′-methyl-2′-butenyl)-2,3,6-trimethyl-phenylacetat in der E-Form, Smp. 144-146 °C, und 0,7 g des entsprechenden Produktes in der Z-Form, Smp. 138-139 °C, isoliert.

Beispiel 3

Herstellung von (E, Z)-4-(2′-5′-Diacetoxy-3′,4′,6′-trimethylphenyl)-2-methyl-2-butenylacetat

5,0 g 2,3,6-Trimethyl-4-(1′-hydroxymethyl-1′-methyl-allyloxy)-phenylacetat (hergestellt nach Beispiel 1) werden in 50 ml Acetanhydrid gelöst und mit 0,1 g Dimethylaminopyridin versetzt. Nach 5 Stunden Rühren bei Raumtemperatur wird das Gemisch während 7 Stunden bei 100 °C gehalten, dann auf Raumtemperatur abgekühlt und auf wässrige Natriumbicarbonatlösung gegossen. Das wässrige Gemisch wird mit Diäthyläther extrahiert, und die Aetherphase wird der Reihe nach mit Natriumbicarbonatlösung und Kochsalzlösung gewaschen, über wasserfreiem Natriumsulfat getrocknet und zur Trockene eingedampft. Es resultieren 5,4 g eines Kristallisates von 4-(2′,5′-Diacetoxy-3′,4′,6′-trimethylphenyl)-2-methyl-2-butenylacetat, welches zu ca. 70 % aus dem E- und zu ca. 30 % aus dem Z-isomeren Produkt besteht. Durch Umkristallisation aux Diisopropyläther kann das reine E-Isomere (Smp. 128-130 °C) gewonnen werden.

**Patentansprüche** (für die Vertragsstaaten : BE, CH, DE, FR, GB, IT, LI, NL)

1. Verfahren zur Herstellung von Hydrochinonderivaten, dadurch gekennzeichnet, dass man eine Verbindung der allgemeinen Formel

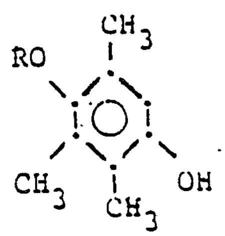

(I)

worin R eine durch Hydrolyse oder oxidativ abspaltbare Hydroxylschutzgruppe bedeutet, mit 3,4-Epoxy-3-methyl-1-buten in Gegenwart eines $d^{10}$-Uebergangsmetall-Katalysators zu einer Verbindung der Formel

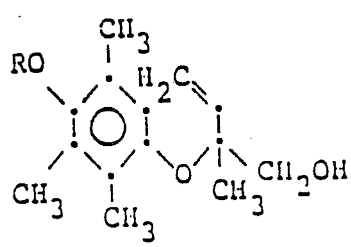

(II)

worin R die obige Bedeutung besitzt, umsetzt.

2. Verfahren nach Anspruch 1, dadurch gekennzeichnet, dass die Hydroxylschutzgruppe R Acetyl ist.

3. Verfahren nach Anspruch 1 oder 2, dadurch gekennzeichnet, dass der $d^{10}$-Uebergangsmetall-Katalysator Bis-(triphenylphosphin)-nickel(O)-dicarbonyl ; der Kupfer(I)-trifluormethansulfonat-Benzol-Komplex ; Tetrakis-(triphenylphosphin)-palladium(O) ; die Verbindung der Formel $Pd^o(diop)_2$, worin diop (2S,3S)-2,3-O-isopropyliden-2,3-dihydroxy-1,4-bis-(diphenylphosphino)-butan oder (2R,3R)-2,3-O-isopropyliden-2,3-dihydroxy-1,4-bis-(diphenylphosphino)-butan ist ; (Trimethylphosphin)-silber(I)-chlorid ; Tetrakis-(triphenylphosphin)-platin(O) ; oder (Triphenylphosphin)-gold(I)-chlorid ist.

4. Verfahren nach einem der Ansprüche 1 bis 3, dadurch gekennzeichnet, dass der $d^{10}$-Uebergangsmetall-Katalysator ein Palladium(O)-enthaltender Komplex ist.

5. Verfahren nach einem der Ansprüche 1 bis 4, dadurch gekennzeichnet, dass die Menge des vorhandenen $d^{10}$-Uebergangsmetall-Katalysators bzw. eines den Palladium(O)-Katalysator erzeugenden Palladium(II)-Salzes 0,05 bis 100 Molprozent, bezogen auf die Menge Verbindung der allgemeinen Formel I, beträgt.

6. Verfahren nach einem der Ansprüche 1 bis 5, dadurch gekennzeichnet, dass man eine erhaltene Verbindung der Formel II einer Claisen-Umlagerung unterwirft, wobei eine Verbindung der allgemeinen Formel

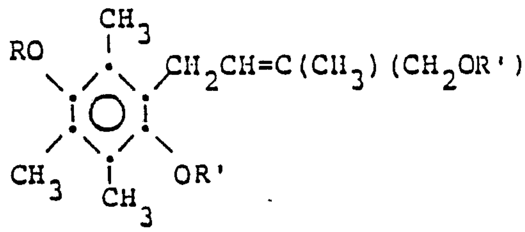

(III)

worin R die oben angegebene Bedeutung besitzt, und R′ Wasserstoff oder — falls die Claisen-Umlagerung in Gegenwart eines Carbonsäureanhydrids oder eines Carbonsäurehalogenids durchgeführt wird — eine Acylgruppe bedeutet, erhalten wird.

7. Verbindungen der allgemeinen Formel

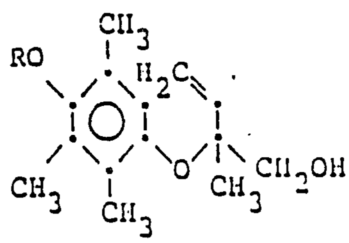

(II)

worin R eine durch Hydrolyse oder oxidativ abspaltbare Hydroxylschutzgruppe bedeutet.

8. Verbindung nach Anspruch 7, dadurch gekennzeichnet, dass die Hydroxylschutzgruppe R Acetyl ist.

9. Verbindungen der allgemeinen Formel

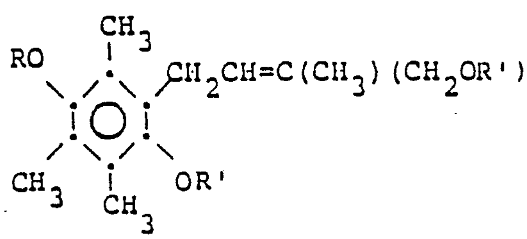

(III)

worin R eine durch Hydrolyse oder oxidativ abspaltbare Hydroxylschutzgruppe und R′ Wasserstoff oder eine Acylgruppe bedeutet, als Isomerengemisch oder in getrennter E- oder Z-Form.

10. Verbindungen nach Anspruch 9, dadurch gekennzeichnet, dass R Acetyl und R′ Wasserstoff oder Acetyl bedeuten.

**Patentansprüche** (für der Vertragsstaat AT)

1. Verfahren zur Herstellung von Hydrochinonderivaten, dadurch gekennzeichnet, dass man eine Verbindung der allgemeinen Formel

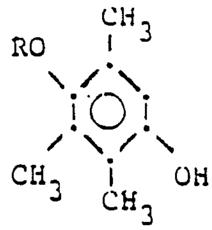

(I)

worin R eine durch Hydrolyse oder oxidativ abspaltbare Hydroxylschutzgruppe bedeutet, mit 3,4-Epoxy-3-methyl-1-buten in Gegenwart eines $d^{10}$-Uebergangsmetall-Katalysator zu einer Verbindung der Formel

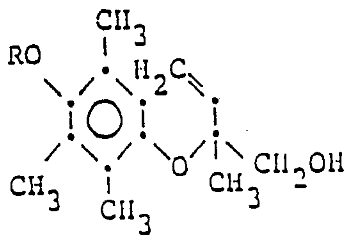

(II)

worin R die obige Bedeutung besitzt, umsetzt.

2. Verfahren nach Anspruch 1, dadurch gekennzeichnet, dass die Hydroxylschutzgruppe R Acetyl ist.

3. Verfahren nach Anspruch 1 oder 2, dadurch gekennzeichnet, dass der $d^{10}$-Uebergangsmetall-Katalysator Bis-(triphenylphosphin)-nickel(O)-dicarbonyl ; der Kupfer(I)-trifluormethansulfonat-Benzol-Komplex ; Tetrakis-(triphenylphosphin)-palladium(O) ; die Verbindung der Formel $Pd^o(diop)_2$, worin diop (2S,3S)-2,3-O-isopropyliden-2,3-dihydroxy-1,4-bis-(diphenylphosphino)-butan oder (2R,3R)-2,3-O-isopropyliden-2,3-dihydroxy-1,4-bis-(diphenylphosphino)-butan ist ; (Trimethylphosphin)-silber(I)-chlorid ; Tetrakis-(triphenylphosphin)-platin(O) ; oder (Triphenyl-phosphin)-gold(I)-chlorid ist.

4. Verfahren nach einem der Ansprüche 1 bis 3, dadurch gekennzeichnet, dass der $d^{10}$-Uebergangsmetall-Katalysator ein Palladium(O)-enthaltender Komplex ist.

5. Verfahren nach einem der Ansprüche 1 bis 4, dadurch gekennzeichnet, dass die Menge des vorhandenen $d^{10}$-Uebergangsmetall-Katalysators bzw. eines den Palladium(O)-Katalysator erzeugenden Palladium(II)-Salzes 0,05 bis 100 Molprozent, bezogen auf die Menge Verbindung der allgemeinen Formel I, beträgt.

6. Verfahren nach einem der Ansprüche 1 bis 5, dadurch gekennzeichnet, dass man eine erhaltene Verbindung der Formel II einer Claisen-Umlagerung unterwirft, wobei eine Verbindung der allgemeinen Formel

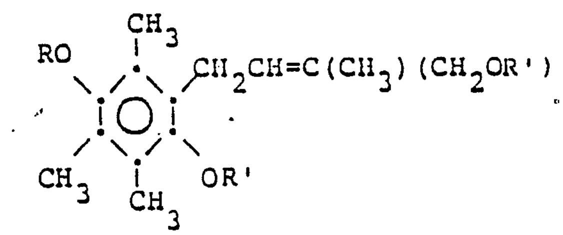

(III)

worin R die oben angegebene Bedeutung besitzt, und R' Wasserstoff oder — falls die Claisen-Umlagerung in Gegenwart eines Carbonsäureanhydrids oder eines Carbonsäurehalogenids durchgeführt wird — eine Acylgruppe bedeutet, erhalten wird.

**Claims** (for the Contracting States : BE, CH, DE, FR, GB, IT, LI, NL)

1. A process for the manufacture of hydroquinone derivatives, characterized by reacting a compound of the general formula

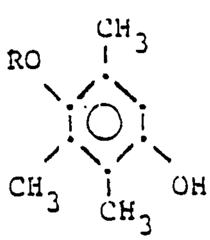

(I)

wherein R signifies a hydroxyl protecting group which is cleavable by hydrolysis or oxidatively, with 3,4-epoxy-3-methyl-1-butene in the presence of a $d^{10}$-transition metal catalyst to give a compound of the formula

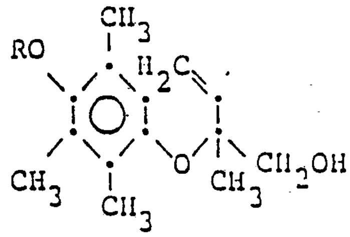

(II)

wherein R has the above significance.

2. A process according to claim 1, characterized in that the hydroxyl protecting group R is acetyl.

3. A process according to claim 1 or 2, characterized in that the $d^{10}$-transition metal catalyst is bis-(triphenylphosphine)-nickel(O)-dicarbonyl ; the copper(I)-trifluoromethanesulphonate-benzene complex ; tetrakis-(triphenylphosphine)-palladium(O) ; the compound of the formula $Pd^o(diop)_2$ in which diop is (2S,3S)-2,3-O-isopropylidene-2,3-dihydroxy-1,4-bis-(diphenyl-phosphino)-butane or (2R,3R)-2,3-O-isopropylidene-2,3-dihydroxy-1,4-bis-(diphenylphosphino)-butane ; (trimethylphosphine)-silver(I) chloride ; tetrakis-(triphenyl-phosphine)-platinum(O) ; or (triphenylphosphine)-gold(I) chloride.

4. A process according to any one of claims 1 to 3, characterized in that the $d^{10}$-transition metal catalyst is a palladium(O)-containing complex.

5. A process according to any one of claims 1 to 4, characterized in that the amount of the $d^{10}$-transition metal catalyst or of a palladium(II) salt producing the palladium(O) catalyst amounts to 0.05 to 100 mol percent based on the amount of compound of general formula I.

6. A process according to any one of claims 1 to 5, characterized in that a compound of formula II obtained is subjected to a Claisen rearrangement, whereby a compound of the general formula

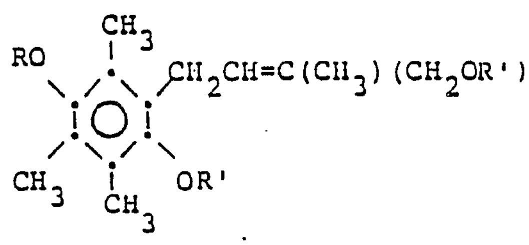

(III)

wherein R has the significance given above and R′ signifies hydrogen or — where the Claisen rearrangement is carried out in the presence of a carboxylic acid anhydride or of a carboxylic acid halide — an acyl group, is obtained.

7. Compounds of the general formula

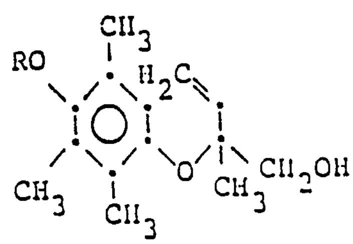

(II)

wherein R signifies a hydroxyl protecting group which is cleavable by hydrolysis or oxidatively.

8. The compound according to claim 7, characterized in that the hydroxyl protecting group R is acetyl.

9. Compounds of the general formula

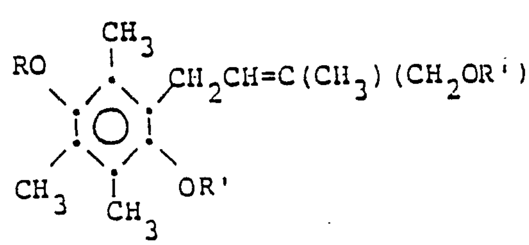

(III)

wherein R signifies a hydroxyl protecting group which is cleavable by hydrolysis or oxidatively and R′ signifies hydrogen or an acyl group, as the isomer mixture or in separated E- or Z-form.

10. Compounds according to claim 9, characterized in that R signifies acetyl and R′ signifies hydrogen or acetyl.

**Claims** (for the Contracting State AT)

1. A process for the manufacture of hydroquinone derivatives, characterized by reacting a compound of the general formula

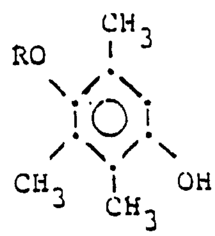

(I)

wherein R signifies a hydroxyl protecting group which is cleavable by hydrolysis or oxidatively, with 3,4-epoxy-3-methyl-1-butene in the presence of a $d^{10}$-transition metal catalyst to give a compound of the formula

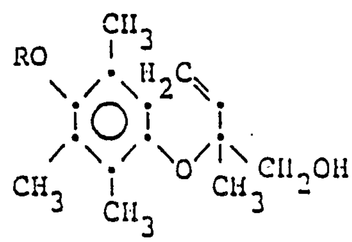

(II)

wherein R has the above significance.

2. A process according to claim 1, characterized in that the hydroxyl protecting group R is acetyl.

3. A process according to claim 1 or 2, characterized in that the $d^{10}$-transition metal catalyst is bis-(triphenylphosphine)-nickel(O)-dicarbonyl ; the copper(I)-trifluoromethanesulphonate-benzene complex ; tetrakis-(triphenylphosphine)-palladium(O) ; the compound of the formula $Pd^{o}(diop)_2$ in which diop is (2S, 3S)-2,3-O-isopropylidene-2,3-dihydroxy-1,4-bis-(diphenyl-phosphino)-butane or (2R, 3R)-2,3-O-isopropylidene-2,3-dihydroxy-1,4-bis-(diphenylphosphino)-butane ; (trimethylphosphine)-silver(I) chloride ; tetrakis-(triphenylphosphine)-platinum(O) ; or (triphenylphosphine)-gold(I) chloride.

4. A process according to any one of claims 1 to 3, characterized in that the $d^{10}$-transition metal catalyst is a palladium(O)-containing complex.

5. A process according to any one of claims 1 to 4, characterized in that the amount of the $d^{10}$-transition metal catalyst or of a palladium(II) salt producing the palladium(O) catalyst amounts to 0.05 to 100 mol percent based on the amount of compound of general formula I.

6. A process according to any one of claims 1 to 5, characterized in that a compound of formula II obtained is subjected to a Claisen rearrangement, whereby a compound of the general formula

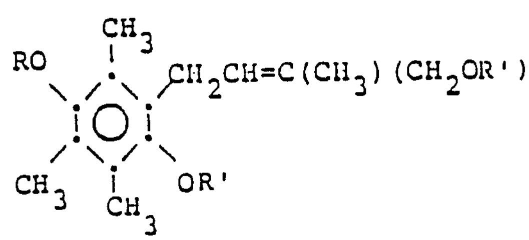

(III)

wherein R has the significance given above and R′ signifies hydrogen or — where the Claisen rearrangement is carried out in the presence of a carboxylic acid anhydride or of a carboxylic acid halide — an acyl group, is obtained.

**Revendications** (pour les Etats contractants : BE, CH, DE, FR, GB, IT, LI, NL)

1. Procédé de préparation de dérivés de l'hydroquinone, caractérisé en ce que l'on fait réagir un composé de formule générale

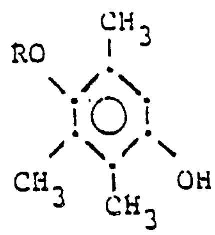

(I)

dans laquelle R représente un groupe protecteur du groupe hydroxy pouvant être éliminé par hydrolyse ou par oxydation, avec le 3,4-époxy-3-méthyl-1-butène, en présence d'un catalyseur à base d'un métal de transition en configuration $d^{10}$, la réaction donnant un composé de formule

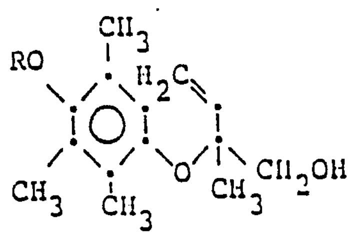

(II)

dans laquelle R a les significations indiquées ci-dessus.

2. Procédé selon la revendication 1, caractérisé en ce que le groupe protecteur R du groupe hydroxy est un groupe acétyle.

3. Procédé selon la revendication 1 ou 2, caractérisé en ce que le catalyseur à base d'un métal de transition en configuration $d^{10}$ est le bis-(triphénylphosphine)-nickel(O)-dicarbonyle ; le complexe trifluorométhane-sulfonate de cuivre (I)-benzène ; le tétrakis-(triphénylphosphine)-palladium(O) ; le composé de formule $Pd^{o}(diop)_2$ dans laquelle diop représente le (2S,3S)-2,3-O-isopropylidène-2,3-dihydroxy-1,4-bis-(diphénylphosphino)-butane ou le (2R,3R)-2,3-O-isopropylidène-2,3-dihydroxy-1,4-bis-(diphénylphosphino)-butane ; le chlorure de (triméthylphosphine)-argent(I) ; le tétrakis-(triphénylphosphine)-plastine(O) ; ou le chlorure de (triphénylphosphine)-or(I).

4. Procédé selon l'une des revendications 1 à 3, caractérisé en ce que le catalyseur à base d'un métal de transition en configuration $d^{10}$ est un complexe contenant du palladium(O).

5. Procédé selon l'une des revendications 1 à 4, caractérisé en ce que la quantité du catalyseur à base d'un métal de transition en configuration $d^{10}$ ou d'un sel de palladium(II) donnant un catalyseur au palladium(O) qui est présente est de 0,05 à 100 moles% par rapport à la quantité du composé de formule générale I.

6. Procédé selon l'une des revendications 1 à 5, caractérisé en ce que l'on soumet un composé obtenu, répondant à la formule II, à une transposition de Claisen, donnant un composé de formule générale

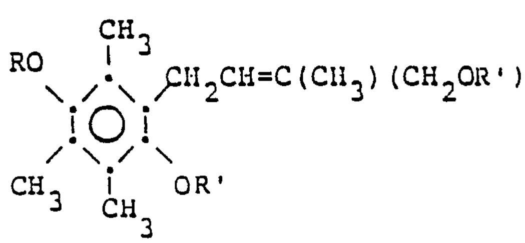

(III)

dans laquelle R a les significations indiquées ci-dessus et R' représente l'hydrogène ou bien — lorsque la transposition de Claisen a été effectuée en présence d'un anhydride d'acide carboxylique ou d'un halogénure d'acide carboxylique — un groupe acyle.

7. Composés de formule générale

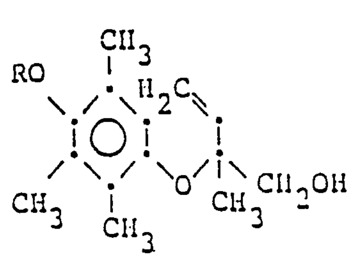

(II)

dans laquelle R représente un groupe protecteur du groupe hydroxy, éliminable par hydrolyse ou par oxydation.

8. Composé selon la revendication 7, caractérisé en ce que le groupe protecteur R du groupe hydroxy est un groupe acétyle.

9. Composés de formule générale

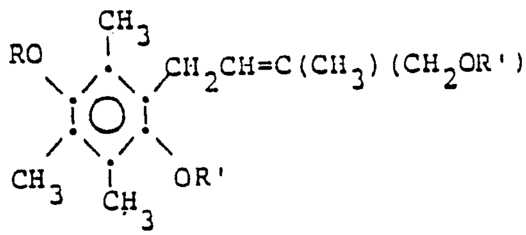

(III)

dans laquelle R représente un groupe protecteur du groupe hydroxy, éliminable par hydrolyse ou par oxydation, et R' représente l'hydrogène ou un groupe acyle, à l'état de mélange d'isomères ou sous les formes E ou Z isolées.

10. Composés selon la revendication 9, caractérisé en ce que R représente un groupe acétyle et R' l'hydrogène ou un groupe acétyle.

**Revendications** (pour l'Etat contractant AT)

1. Procédé de préparation de dérivés de l'hydroquinone, caractérisé en ce que l'on fait réagir un composé de formule générale

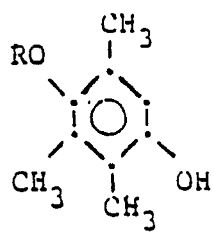

(I)

dans laquelle R représente un groupe protecteur du groupe hydroxy pouvant être éliminé par hydrolyse ou par oxydation, avec le 3,4-époxy-3-méthyl-1-butène, en présence d'un catalyseur à base d'un métal de transition en configuration $d^{10}$, la réaction donnant un composé de formule

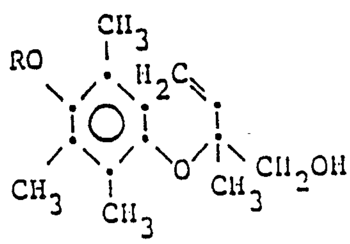

(II)

dans laquelle R a les significations indiquées ci-dessus.

2. Procédé selon la revendication 1, caractérisé en ce que le groupe protecteur R du groupe hydroxy est un groupe acétyle.

3. Procédé selon la revendication 1 ou 2, caractérisé en ce que le catalyseur à base d'un métal de transition en configuration $d^{10}$ est le bis-(triphénylphosphine)-nickel(O)-dicarbonyle ; le complexe trifluorométhane-sulfonate de cuivre(I)-benzène ; le tétrakis-(triphénylphosphine)-palladium(O) ; le composé de formule $Pd^{o}(diop)_2$ dans laquelle diop représente le (2S,3S)-2,3-O-isopropylidène-2,3-dihydroxy-1,4-bis-(diphénylphosphino)-butane ou le (2R,3R)-2,3-O-isopropylidène-2,3-dihydroxy-1,4-bis-(diphénylphosphino)-butane ; le chlorure de (triméthylphosphine)-argent(I) ; le tétrakis-(triphénylphosphine)-platine(O) ; ou le chlorure de (triphénylphosphine)-or(I).

4. Procédé selon l'une des revendications 1 à 3, caractérisé en ce que le catalyseur à base d'un métal de transition en configuration $d^{10}$ est un complexe contenant du palladium(O).

5. Procédé selon l'une des revendications 1 à 4, caractérisé en ce que la quantité du catalyseur à base d'un métal de transition en configuration $d^{10}$ ou d'un sel de palladium(II) donnant un catalyseur au palladium(O) qui est présente est de 0,05 à 100 moles% par rapport à la quantité du composé de formule générale I.

6. Procédé selon l'une des revendications 1 à 5, caractérisé en ce que l'on soumet un composé obtenu, répondant à la formule II, à une transposition de Claisen, donnant un composé de formule générale

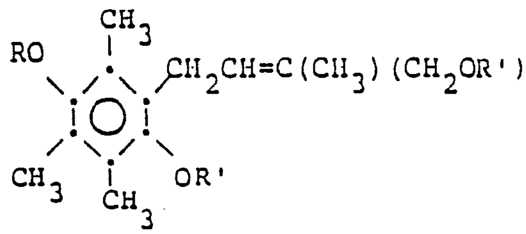

(III)

dans laquelle R a les significations indiquées ci-dessus et R' représente l'hydrogène ou bien — lorsque la transposition de Claisen a été effectuée en présence d'un anhydride d'acide carboxylique ou d'un halogénure d'acide carboxylique — un groupe acyle.